# EUROPEAN PATENT APPLICATION

(11) **EP 2 179 747 A1**
(43) Date of publication of application: **28.04.2010**
(21) Application number: 08167580.3
(22) Date of filing: 27.10.2008
(51) Int. Cl.: A61L 9/01

(54) **Method of reducing malodors**

(71) Applicant: THE PROCTER & GAMBLE COMPANY, Cincinnati, Ohio 45202 (US)
(72) Inventor: Cornelis, Sylvia, 1853 Strombeek-Bever (BE); Delplancke, Patrick Firmin August, 9270 Laarne (BE); Garmyn, Wim, 2018 Antwerpen (BE); Sarcinelli, Luca, 00052 Cerveteri (Rome) (IT); Scialla, Stefano, 00128 Rome (BE)
(74) Representative: Kellenberger, Jakob

(57) **Abstract**

A method of reducing the formation of malodors in a confined space. More specifically, it is provided a method of reducing the formation of malodors in a confined space, comprising the step of placing a moisture-permeable, water-insoluble sachet comprising a solid composition comprising a hypohalite bleach and/or a peroxygen bleach in close proximity of a source of humidity present within said confined space.

## Description

### FIELD OF THE INVENTION

The present invention relates to a method of reducing the formation of malodors in a confined space, preferably confined space that is prone to have relatively high humidity, more preferably in a bathroom. More specifically, the present invention is directed to a method of reducing the formation of malodors comprising the step of placing a sachet comprising a solid composition comprising a hypohalite bleach and/or a peroxygen bleach in selected locations of a confined space, preferably confined space that is prone to have relatively high humidity, more preferably a bathroom.

### BACKGROUND OF THE INVENTION

Air freshener or deodorizing systems have long been sought by consumers, in both residential and commercial environments. In an attempt to meet the demand for air fresheners or deodorizers, numerous products have been developed and are presently available in the martketplace. In general, these prior art products are sold as solids, liquids, gel or aerosol sprays to provide the desired effect. Typically, these prior art products are used to eliminate, chemically change, or mask an existing odor. Products known in the art typically work by absorbing odorous molecules, dissolving or emulsifying such molecules, or killing bacteria that cause the offensive odor.

Among the variety of offensive odors and the variety of spaces where such malodors might be generated, confined spaces are particularly prone to develop unwanted odors. In the context of household applications, confined spaces comprising a source of humidity, such as bathrooms, are particularly subject to development of malodors.

Hypohalite and Peroxygen bleaches have long been recognized as a disinfecting and sterilizing agent in hard surface and fabric cleaning and disinfecting applications. However, such bleach species have so far not been employed in air freshener or deodorizing systems.

The problem of providing long-term deodorizing or air freshening is particularly critical when trying to install a pleasant odor in areas or environments in which offensive odors continuously exist, such as in confined spaces comprising a source of humidity, like bathrooms.

It is therefore an objective of the present invention to provide a method of reducing the formation of a broad variety of malodors which are particularly prone to develop in a confined space, preferably a confined space that is prone to have relatively high humidity, more preferably in a bathroom.

Advantageously, the method according to the present invention is capable of providing long-term and sustained malodors reduction or air freshening. Also the method of the present invention is able to provide malodor reduction or air freshening in a highly controlled manner without requiring any intervention of the user. A further advantage associated with the method according to the present invention is that it involves very simple and inexpensive material, and may be easily employed in both residential and commercial establishments for providing malodors reduction or air-freshening in confined spaces, preferably a confined spaces that is prone to have relatively high humidity, more preferably in a bathrooms. It is still a further advantage that the method of the present invention is completely safe for use by humans.

Other advantages and more specific properties of the method according to the present invention will be clear after reading the following description of the invention.

### SUMMARY OF THE INVENTION

The present invention relates to a method of reducing the formation of malodors in a confined space, comprising the step of placing a moisture-permeable, water-insoluble sachet comprising a solid composition comprising a hypohalite bleach and/or a peroxygen bleach in close proximity of a source of humidity present within said confined space.

In another embodiment, the present invention is directed to a method of freshening air in a confined space, the method comprising the step of placing a moisture-permeable, water-insoluble sachet comprising a solid composition comprising a hypohalite bleach and/or a peroxygen bleach in close proximity of a source of humidity present in the confined space.

The present invention further encompasses the use of a moisture-permeable, water-insoluble sachet comprising a solid composition comprising a hypohalite bleach and/or a peroxygen bleach, for reducing the formation of malodors in a confined space

### DETAILED DESCRIPTION OF THE INVENTION

### Sachet

The method according to the present invention involves using a moisture-permeable, water-insoluble sachet. The sachet for use herein may be formed from any material well known in the art of functional sachets for being moisture-permeable and water-impervious. Typically, the sachet for use herein may be defined as being formed from a microporous membrane comprising one or more selected from the group consisting of woven or non-woven, synthetic or natural fibers integrally joined together, wood pulp, and plastic films or sheets. Preferably, the sachet for use in the present invention is formed from a polymeric plastic sheet comprising one selected from the group consisting of polyurethanes, polyethers, polyesters, polypropylenes, polystyrenes, and combinations thereof.

In an even more preferred embodiment, the sachet for use herein is made from materials commercially available under the tradenames TYVEK® and GORTEX®, most preferably from TYVEK®. Such particular materials enable water vapor and ambient air to enter into the sachet while said material being substantially impervious to water.

Suitable sachet may have any suitable configuration, form or dimension for accommodating the solid composition herein. Suitable sachet for use in the present invention will easily be recognized by those skilled in the art. In a preferred embodiment the sachet for use herein has a substantially rectangular shape, preferably a rectangular shape, although the present invention is not so limited.

However, it will be easily apparent to those skilled in the art that, depending upon the particular aesthetic impression which is ultimately aimed at, other configurations and shapes of the sachet may be used.

According to an alternative embodiment of the present invention, the sachet for use herein may be incorporated within a suitable container which may in turn be provided with aesthetic features. Suitable containers for use herein will be easily recognized by those skilled in the art. Typically, such containers shall be provided with suitable openings as required for the ambient air to diffuse through said containers. As a way of example, suitable containers include but are not limited to, a box, a bottle, a pouch, an envelope, a can, a tube, and a bag.

The sachet herein preferably comprises less than 200 grams, preferably less than 100 grams, more preferably from 10 grams to 95 grams, and most preferably from 50 grams to 95 grams of the solid composition herein.

### Solid Composition

The sachet according to the present invention comprises a solid composition.

Preferably, the solid composition herein is in the form of : a powder composition, a granular composition; or even in the form of a tablet, preferably of compressed powder and/or granular solid composition.

In a highly preferred embodiment according to the present invention, the composition herein is a powder composition.

The solid composition according to the present invention can be prepared in several ways which will be easily recognized by those skilled in the art. The preferred method is to prepare in a dry atmosphere an intimate physical mixture of fine powders of the constituents having particle sizes preferably below 200 µm. However, larger particles may also be used.

### Bleach species

The solid composition herein comprises a hypohalite bleach and/or a peroxygen bleach. The bleaches herein are solid bleaches, which are preferable water-soluble.

The solid composition preferably comprises 100% hypohalite bleach and/or a peroxygen bleach. In a preferred embodiment, wherein optional ingredients are present in said composition the solid comprising comprises from 2% to 100%, preferably from 2% to 50% and most preferably from 2% to 10% by weight of the solid compositions of the hypohalite bleach and/or a peroxygen bleach.

### Hypohalite bleach

Hypohalite bleaches may be provided by a variety of sources, including bleaches that are oxidative bleaches and subsequently lead to the formation of positive halide ions as well as bleaches that are organic based sources of halides such as chloroisocyanurates.

Suitable hypohalite bleaches for use herein include the alkali metal and alkaline earth metal hypochlorites, hypobromites, hypoiodites, chlorinated trisodium phosphate dodecahydrates, potassium and sodium dichloroisocyanurates, potassium and sodium trichlorocyanurates, N-chloroimides, N-chloroamides, N-chloroamines and chlorohydantoins.

In another preferred embodiment said hypohalite bleach herein is selected from the group consisting of : an alkali metal or alkaline earth metal hypochlorite; chlorinated trisodium phosphate dodecahydrate; potassium dichloroisocyanurate; sodium dichloroisocyanurate; potassium trichlorocyanurate; and sodium trichlorocyanurate; and a mixture thereof.

More preferably, said hypohalite bleach herein is selected from the group consisting of : an alkali metal or alkaline earth metal hypochlorite selected from the group consisting of lithium hypochlorite and calcium hypochlorite; chlorinated trisodium phosphate dodecahydrate; potassium dichloroisocyanurate; sodium dichloroisocyanurate; potassium trichlorocyanurate; and sodium trichlorocyanurate; and a mixture thereof.

Even more preferably, said hypohalite bleach herein is sodium dichloroisocyanurate and/or calcium hypochlorite.

### Peroxygen bleach

A suitable peroxygen bleach herein is a source of hydrogen peroxide. As used herein a hydrogen peroxide source refers to any compound which produces perhydroxyl ions when said compound is in contact with water.

Suitable water-soluble sources of hydrogen peroxide for use herein include percarbonates, persilicate, persulphate such as monopersulfate, perborates, preformed peroxyacids, alkyl hydroperoxides, peroxides, aliphatic diacyl peroxides, and urea peroxide, and mixtures thereof.

Suitable preformed peroxyacids for use in the compositions according to the present invention include diperoxydodecandioic acid DPDA, magnesium perphthalatic acid, perlauric acid, perbenzoic acid, diperoxyazelaic acid and mixtures thereof.

Suitable hydroperoxides for use herein are tert-butyl hydroperoxide, cumyl hydroperoxide, 2,4,4-trimethylpentyl-2-hydroperoxide, di-isopropylbenzene-monohydroperoxide, tert-amyl hydroperoxide, 2,5-dimethyl-hexane-2,5-dihydroperoxide or mixtures thereof.

Suitable aliphatic diacyl peroxides for use herein are dilauroyl peroxide, didecanoyl peroxide, dimyristoyl peroxide or mixtures thereof.

It has been found that upon exposure of the sachet herein to water vapor in air, the bleach species present in the solid composition herein are released in the form of a vapour. In such vapor form, the bleach species decomposes air-borne malodors and thereby provides an air deodorizing activity.

By "vapor form" it is meant herein that the bleach species is dissolved in water vapour, which acts as a carrier to transport the bleach species (e.g., hypochlorous acid) outside the sachet. Indeed, water vapor present in the ambient air (coming from the relative humidity of the surrounding air) passes through the sachet and upon contact with the bleach species partially or full dissolves said bleach species.

Also, the generation of the bleach in vapor form herein need not be at a constant rate. It is permissible to have a fluctuating rate.

The generation of the bleach vapor herein may last for a sustained period of time, i.e. the bleach species will be vaporized during a short period of time (e.g. several minutes) to a long period of time spanning several hours or weeks. The length of the sustained period of time will depend upon the relative amounts of the constituents in the mixture.

According to a preferred embodiment of the invention, the generation of the bleach vapor herein will vary depending on the relative humidity of the surrounding atmosphere, the ratio of the reactants, the diluent gas flow rate (e.g. air) through the treated space, and the ratio of the amount of bleach material to the volume of the treated space. Generally, the higher the relative humidity the higher rate of production of the bleach vapor.

It will be understood that for a given unit of the solid composition represented by a unitary sachet, a sustained amount of bleach vapor will be produced. For some specific applications, it may be desirable to employ multiple units of the mixture to achieve the desired deodorizing effect.

In the practice of the present invention, the relative humidity of the atmosphere to which the solid composition is exposed during use can range from low humidity (e.g. 10% relative humidity) up to 100% relative humidity conditions.

The kit of the present invention may be used for a variety of commercial applications. The kits herein may also be used to treat spaces comprising diverse items such as animal waste; pet litters; garbage bins; laundry baskets; medical devices; food products including meats, vegetables, fruit, grain and nuts; as well as items made from fabrics including drapes, upholstery, and clothes. Examples of environments which may be treated include those containing noxious and/or objectionable gases such as animal environments, smoke-laden environments (tobacco smoke), and exhaust systems from noxious gas producing facilities (e.g. chemical plants).

In the preferred embodiment of the present invention the sachet herein is particularly suitable for deodorizing confined spaces which are prone to have relatively high humidity such as bathroom, kitchens, cellars, closets and shoes. It has been indeed discovered that confined spaces with relative high humidity are particularly prone to develop malodors.

### OPTIONAL FEATURES

The solid composition herein my comprise an optional ingredient selected from the group consisting of : fillers; pH-adjustment agents; and dehydrating agents; and mixtures thereof.

Suitable fillers for use herein include but are not limited to salts with a carbonate-, sulphate- or chloride-anionic group with sodium, calcium, potassium or magnesium as counterions.

Suitable pH adjusting agents include water-soluble acids and bases.

The composition in accordance with the present invention may optionally comprise at least one dehydrating agent which is primarily intended to absorb water to minimize or eliminate an initial brief duration activation of the bleach species due to residual water vapor present in the atmosphere when the mixture is packaged within the moisture permeable, water-impervious sachet.

Also, it has been surprisingly discovered that the combination of the bleach herein together with a dehydrating agent contributes to obtain improved deodorizing effect. The optional use of dehydrating agent to minimize bleach dissolution in the sachet upon storage can ensure that the mixture will react for the longest period of time when exposed to water vapor under operating conditions. The presence of dehydrating agent may delay the desired onset of bleach dissolution when the mixture is exposed to water vapor. Therefore, the length of time of the reaction is also dependent, in part, on how much water vapor is present in the atmosphere contained within the sachet.

Alternatively, said dehydrating agent may be kept separate from said moisture-permeable, water-impervious sachet. According to this alternative embodiment, the kit according to the present invention further comprises a dehydrating agent as a distinct element.

Suitable dehydrating agents for use herein include but are not limited to, activated calcium chloride, activated calcium sulfate, activated zeolite X, activated zeolite A, activated bentonite clay, activated silica gel, activated attapulgite, and mixtures thereof. The term "activated' means that the particular material has been substantially dehydrated, for example, by heating at 300C for one hour. The total amount of dehydrating agent may vary depending on several factors, for example, the ambient humidity when the material is packaged into the suitable sachet, the water permeability of the sachet material and the desired shelf life of the product. Typically, the dehydrating agent is present in a total amount from 0.1% to 25% by weight based o the total weight of the solid composition.

The method of the present invention may optionally, but preferably, involve the use of a perfume composition. It has been indeed discovered that a number of hypohalite and peroxygen bleach species are offending to the olfactory senses and may even be perceived by consumers as unacceptable because of its unpleasant odor, this applies especially to the former group of bleach species. This unrecognized problem may detrimentally affect the overall deodorizing benefit provided by the method of the present invention. In addition, consumers prefer air freshener or deodorizing systems that in addition to deodorizing or air freshening also provide an agreeable fragrance to the ambient air.

As used herein the term "perfume" is used to indicate any odoriferous material that is subsequently released into the ambient air. The perfume will most often be liquid at ambient temperatures. A wide variety of chemicals are known for perfume uses, including materials such as aldehydes, ketones, and esters. More commonly, naturally occurring plant and animal oils and exudates comprising complex mixtures of various chemical components are known for use as perfumes. The perfumes herein can be relatively simple in their compositions or can comprise highly sophisticated complex mixtures of natural and synthetic chemical components, all chosen to provide any desired odor. Typical perfumes can comprise, for example, woody/earthy bases containing exotic materials such as sandalwood, civet and patchouli oil. The perfumes can be of a light floral fragrance, e.g. rose extract, violet extract, and lilac. The perfumes can also be formulated to provide desirable fruity odors, e.g. lime, lemon, and orange. Likewise, the perfumes delivered in the compositions and articles for use in the method of the present invention may be selected for an aromatherapy effect, such as providing a relaxing or invigorating mood. As such, any material that exudes pleasant or otherwise desirable odors can be used as a perfume active in the method of the present invention. Perfume materials are described more fully in S. Arctander, Perfume Flavors and Chemicals. Vols. I and II. Aurthor, Montclair, N.J., and the Merck Index, 8th Edition, Merck & Co., Inc. Rahway, N.J.

According to the present invention, perfume composition for use herein may either be part of the moisture-permeable, water-impervious sachet or alternatively may be kept separate from said sachet.

In the specific embodiment of the invention wherein the perfume composition is part of the sachet, the composition may be loaded onto the external surface of the sachet or onto the internal surface of the sachet. Suitable techniques for loading said perfume composition onto the sachet will be easily recognized by the skilled person. For example, perfume composition may be incorporated directly within the material used to form such sachet, or may applied onto the surface of said sachet by means of printing or spraying.

Alternatively, said perfume composition may be incorporated in the solid composition inside said sachet. In such specific embodiment of the invention, the perfume composition may be selected from any perfume composition commonly known as being compatible with oxidizing agents. Suitable perfume composition for use according to this particular embodiment of the invention may be easily determined by those skilled in the art of perfumery. As a way of example, suitable perfume compositions are described e.g. in US-B1-6,255,268 on column 6 line 10 to column 9 line 50. Other examples of suitable perfume compositions are disclosed in EP-B1-0622451 on column 3 lines 22-50.

According to a preferred execution however, the perfume composition for use in the context of the present invention is kept separate from said moisture-permeable, water-impervious sachet. In accordance with this specific embodiment, suitable perfume composition may be selected from any perfume composition commonly known in the art of perfumery including perfume composition know to be chemically incompatible with oxidizing gases. It will be understood that one of ordinary skill in the art, may easily recognize suitable perfume composition for use in the present invention. Typically, the perfume composition for use in accordance with this specific embodiment is a mixture of organic compounds admixed so that the combined odors of the individual components produce a pleasant or desired fragrance. Suitable perfume composition for use in accordance with the present embodiment may take any physical form including solid, particulate, gel, liquid, paste, tablet, bar, gas, and mixtures thereof. In a preferred embodiment, the perfume composition for use herein is in the form of liquid, solid, gel, adhesive gel, paste, tablet, and mixtures thereof.

In a preferred execution of the present invention, the perfume composition for use herein is a polymeric composition obtained by combining a low melting point polyamide polymer with a polar thermoplastic elastomer and a perfume. Such polymeric compositions are described for example in US-A1-2006/0099168.

The term "low melting point polyamide polymers" includes all polyamides having a melting point below 130°C, preferably below 110°C, more preferably below 100°C. Typically and preferably, the low melting point polyamides for use in the present invention are solid at room temperature. Preferred polyamides are terminated polyamides, particularly preferred are ester terminated polyamides. Examples of these low melting point polyamides include those marketed by Arizona chemicals under the trade name of SYLVACLEAR®.

The term "polar thermoplastic elastomer" includes multiphase polymers that comprise "hard" and "soft" phases chemically bonded together in the polymer chain. The "hard" phase is solid at room temperature and flows upon heating. Examples include blocks of amide, ester and urethane groups. The "soft" phase is rubbery at room temperature. Examples include polyether blocks such as poly(ethylene glycol), poly(propylene glycol) or poly(tetramethylene glycol). At room temperature, the presence of the "hard" phases in the polymer imparts strength and good mechanical properties. When the polymer is heated, these phases become liquid and the polymer melts, allowing for processing in the molten state. Upon re-cooling to room temperature, the phases solidify and the good mechanical properties are regained. A comprehensive definition of thermoplastic elastomers can be found in Vol 9 of the Kirk-Othmer Encyclopedia of Chemical Technology (4th Edition - Wiley- Interscience, 1996) - under the voice "Elastomers", subvoice "Thermoplastic Elastomers". Among these polymers those which are suitable for the present invention are those comprising at least one polar monomer. Polar monomers are those monomers which comprise at least a C-X linkage in the molecule wherein said C-X linkage is a polar linkage. Preferably X is an N, S, F, Cl or O atom. More preferably said polar linkage is part of a carbonyl group and, more preferably, of an ester group. Preferred polar monomers for the present invention are vinyl acetate, vinyl alcohol, methyl acrylate, ethyl acrylate, butyl acrylate, acrylic acid and salts formed therefrom, methacrylic acid and salts formed therefrom, maleic anhydride, glycidyl methacrylate and carbon monoxide. More preferably the hard phases preferably comprise blocks of amide, ester or urethane groups and the soft phases preferably comprise polyether blocks. Examples of these polar thermoplastic elastomers include thermoplastic polyurethanes, such as those produced under the trade names ESTANE® by Noveon, and PELLETHANE® by Dow Chemicals; thermoplastic polyesters, also known as polyether ester copolymers, such as those produced under the trade names HYTREL® by Dupont and ARNITEL® by DSM, and thermoplastic polyamides, also known as polyether amide copolymers, such as those produced under the trade name PEBAX® by Atofina.

According to a highly preferred execution of the present invention, the perfume composition for use herein is a polymeric composition obtained by combining an ester terminated polyamide, a thermoplastic polyether amide copolymer and a perfume, according to the method described in US-A1-2006/0099168.

Preferably, the perfume composition is provided with an aesthetically appealing shape or form. Depending upon the ultimately desired aesthetic effect, the perfume composition may alternatively be comprised within a distinct container which may in turn be provided with aesthetic features. Suitable containers for use herein will be easily recognized by those skilled in the art of perfumery. Typical containers include but are not limited to, a box, a bottle, a pouch, an envelope, a can, a tube, beads, flakes and bags.

Typically, such containers shall be provided with suitable openings as required for perfume composition to diffuse through said containers. Such openings will also allow free circulation of air within said containers.

According to the present invention, the Applicant has surprisingly discovered that by providing a method involving the use of a perfume composition, the potential user is not only not overcome by the unpleasant odor associated with the a number of hypohalite and peroxygen bleach species (especially applicable to the hypohalite bleaches) but also provided with a means to provide agreeable fragrance to the ambient air. The present invention therefore provides the user with a more pleasant deodorizing experience since the method according to the invention does not only neutralize malodors which may be present in a confined space, preferably a confined space that is prone to have relatively high humidity, more preferably in a bathroom, but also delivers a desired fragrance unaffected by any other unwanted side malodors.

As a further optional feature, the water-insoluble sachet for use in the method of the invention may comprise a visual means indicating when the water-insoluble sachet needs to be replaced. Suitable visual means for use herein may be any such visual means commonly known in the art of functional sachets. Typical visual means for use herein comprise but are not limited to badges or patches based upon color-changing or degrading material technologies. Preferably, the visual means is selected to be a patch based upon color-changing technology.

### METHOD OF REDUCING THE FORMATION OF MALODORS

The present invention is primarily directed to a method of reducing the formation of malodors in a confined space, preferably confined a space that is prone to have relatively high humidity, more preferably in a bathroom, comprising the step of placing a moisture-permeable, water-insoluble sachet comprising a solid composition comprising a hypohalite bleach and/or a peroxygen bleach in close proximity of a source of humidity within said confined space.

The method of the present invention may find its use in any confined spaces which may be present in residential and commercial establishments or within industrial facilities. In a preferred embodiment, the method of the invention is particularly suited for confined spaces which are prone to have relatively high humidity. As way of example, such confined spaces include but are not limited to, bathroom, toilets, shower cabins, indoor swimming pools, boats, kitchens, cellars, closets, lockers, shoes and cars. In a preferred embodiment, the method according to the invention is used in confined spaces selected from bathroom, toilets and shower cabins. Most preferably, the method of the invention is used to reduce formation of malodors in a bathroom.

By "a source of humidity" it is meant herein any source of water vapor in said space, such as a tap, basin, pool, toilet, shower, bathtub, sewer, drainage openings, water or steam pipes, surface prone to formation of water condensation, etc.

In a preferred embodiment, wherein the confined space herein is a bathroom, the moisture-permeable, water-insoluble sachet herein is placed in close proximity of a bathtub and/or shower present in said bathroom.

By "close proximity", it is meant herein that the moisture-permeable, water-insoluble sachet is placed at a distance not exceeding 3 meters, preferably not exceeding 2 meters, more preferably not exceeding 1 meter, in any directions from said source of humidity (preferably bathtub and/or shower present in the bathroom).

The method of the present invention may be useful for reducing the formation of a broad variety of malodors which may particularly develop in confined spaces, preferably in confined spaces that are prone to have relatively high humidity more preferably in bathrooms. Typical examples of such malodors include, but are not limited to mold and mildew odors, odors from soap scum and body soil residues, pipe malodors, smoke; cleaning or personal care products malodors, such as odors from garbage bins and laundry baskets, and pet litters. More specifically, the method according to the present invention is particularly adapted for reducing mold and mildew odors.

In the context of the present invention, it has surprisingly discovered that by placing a moisture-permeable, water-insoluble sachet as above described in close proximity of a source of humidity (such as a bathtub and/or shower) present in a confined space, preferably confined space that is prone to have relatively high humidity, more preferably in a bathroom, enhanced reduction of malodors is achieved when compared to the situation where such water-insoluble sachet is placed in any other locations within said confined space. The present invention is based on the discovery that specific locations in confined spaces, which are characterized by a relative high humidity, are particularly prone to develop malodors. As above indicated, the amount of dissolved bleach generated by the water-insoluble sachet for use herein will depend, for a large part, on the relative humidity of the surrounding atmosphere. More specifically, the higher humidity will result in a higher the level of bleach dissolution. In other words, the method according to the present invention permits a targeted, controlled and adaptable action towards reducing the formation of malodors in a confined space, preferably confined space that is prone to have relatively high humidity, more preferably in a bathroom.

According to the method of the invention, the moisture-permeable, water-insoluble sachet may be put down on a flat surface or may alternatively hang on a hook or similar holder. Preferably, the sachet is hanged up on a hook. According to this preferred execution of the invention, it is believed that improved water vapor circulation within said sachet is achieved and therefore enhanced reduction of the formation of malodors is consequently obtained. It is understood that the person skilled in the art as well as the potential user will easily determine the most suitable locations for placing the water-insoluble sachet, in order to achieve the desired deodorizing benefit.

Also, depending upon the intensity of the malodors to be reduced, the user may find useful to place one or more units of said moisture-permeable, water-insoluble sachet in a confined space, preferably confined space that is prone to have relatively high humidity, more preferably in a bathroom to be treated.

Accordingly, the articles for use in the context of the present invention may optionally comprise specific instructions which may help the user to select the number of sachets to use depending upon the confined space, preferably the confined space that is prone to have relatively high humidity, more preferably the bathroom, and the specific malodors to be treated and to choose specific suitable locations for said sachet within said confined space.

Such set of usage instructions for selecting and dosing the sachet and/or the optional articles for use in the present invention, may be provided in any suitable locations such as in a pamphlet, a computer screen, a printed ticket, a kiosk, a sign, a product container, an advertisement, a product display, an Internet website, a video, and a combination thereof. Preferably the set of usage instructions are provided on the sachet container, a product display, or a combination thereof, as these locations are easy to reference. More preferably, the set of usage instructions are provided on the sachet container, as the set of usage instructions is thus unlikely to become lost and/or separated from the sachet when it is needed. Alternatively, the set of usage instructions are provided on any of the optional articles for use herein. Without intending to be limited by theory, it is believed that such set of usage instructions may significantly reduce misuse and/or inappropriate use by the consumer of the sachet and/or the different optional articles for use in the present invention. Also, the set of usage instruction may help the user achieving an improved deodorizing effect.

Incidentally, and according to another embodiment of the present invention, it is provided a method of reducing humidity in a confined space, preferably confined space that is prone to have relatively high humidity, more preferably in a bathroom, due to the water-vapor absorbing properties of the water-insoluble sachet for use in the present invention.

The method according to the invention provides the additional benefit that the treated confined spaces, preferably confined spaces that is prone to have relatively high humidity, more preferably bathrooms, are kept fresher since the ambient humidity is partially absorbed by the solid composition within the water-insoluble sachet for use in the present invention. Therefore, the method according to the present invention may incidentally operate as a method for partially dehumidifying a confined space, preferably confined space that is prone to have relatively high humidity, more preferably a bathroom.

### USE OF THE SACHET FOR REDUCING FORMATION OF MALODORS

The present invention is further directed to the use of a moisture-permeable, water-insoluble sachet comprising a solid composition comprising a hypohalite bleach and/or a peroxygen bleach, for reducing the formation of malodors in a confined space, preferably confined space that is prone to have relatively high humidity, more preferably in a bathroom.

### EXAMPLES

### Examples 1:

Moisture-permeable, water-impervious sachets of rectangular shape made of GORETEX® material is filled respectively with between 20 and 200 grams of solid compositions I to VI (amount as indicated below). The compositions were made by mixing the listed ingredients in listed proportions. All proportions are % by weight of the total composition.

| Compositions (weight%): | | | | | | |
|---|---|---|---|---|---|---|
| | I | II | III | IV | V | VI |
| 2Na₂CO₃.H₂O₂ | - | - | - | 20.0 | 9.0 | 5.0 |
| Ca(ClO)₂ | 5.0 | 9.0 | 20.0 | - | - | - |

| Compositions (weight%): | | | | | | |
|---|---|---|---|---|---|---|
| NaCl | 75.0 | 30.0 | - | - | 26.0 | 40.0 |
| Na₂SO₄ | - | 30.0 | - | 79.0 | 65.0 | 40.0 |
| Na₂CO₃ | 20.0 | 30.0 | 79.9 | - | - | 14.5 |
| Perfume | - | 1.0 | 0.1 | 1.0 | - | 0.5 |
| Amount used [grams] | 20 | 200 | 150 | 100 | 150 | 50 |

### Testing:

A perfume composition in solid form (200 grams) is obtained by charging 70 parts of Lavender natural extract into a vessel (sealed or under reflux) together with 10 parts of sucrose acetate isobutyrate (SAIB from Eastman Chemical) as plasticizer and mixed at room temperature. The temperature is then elevated to 80°C. 10 parts of Pebax® 2533 (from Total Fina) as polar thermoplastic elastomer and 10 parts of low melting point polyamide Sylvaclear® AF 1900 from Arizona Chemical are charged into the vessel and stirred till complete dissolution. The composition is then let to cool down and solidify at room temperature.

For each sachet obtained under Examples 1, containing compositions I to VI, a solid perfume composition as described above, is placed on a flat surface at about 1 meter from said water-impervious sachet in a typical bathroom environment. The formation of malodors, for instance mold and mildew malodors, is reduced for about 1 month and no bleach species odor is noticeable during that period. Also, ambient air is perceived as fresher due to the reduction of persistent humidity inside the treated bathroom.

### Example 2 :

A sachet as described above comprising Composition I and a perfume composition (200 grams) as described herein above under Testing are placed in distinct cardboard-made containers provided with openings. Both containers are hanged to the roof of a regular basement at about 1 meter distance from each other. The formation of malodors, for instance mold and mildew malodors, are reduced for about 1 month and no bleach species odor is noticeable during that period. Also, ambient air is perceived as fresher due to the reduction of persistent humidity inside the treated basement.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A method of reducing the formation of malodors in a confined space, comprising the step of placing a moisture-permeable, water-insoluble sachet comprising a solid composition comprising a hypohalite bleach and/or a peroxygen bleach in close proximity of a source of humidity present within said confined space.

2. A method according to claim 1 wherein said solid composition comprises a hypohalite bleach, preferably a hypohalite bleach is selected from the group consisting of : an alkali metal or alkaline earth metal hypochlorite; chlorinated trisodium phosphate dodecahydrate; potassium dichloroisocyanurate; sodium dichloroisocyanurate; potassium trichlorocyanurate; and sodium trichlorocyanurate; and a mixture thereof.

3. A method according to claim 1 wherein said solid composition comprises a peroxygen bleach, preferably a water-soluble source of hydrogen peroxide, preferably selected from the group consisting of : percarbonates, persilicate, persulphate such as monopersulfate, perborates, preformed peroxyacids, alkyl hydroperoxides, peroxides, and urea peroxide; and aliphatic diacyl peroxides and mixtures thereof.

4. A method according to any of the preceding claims wherein said mixture further comprises a dehydrating agent.

5. A method according to claim 4 wherein said mixture comprises from 0.1 wt% to 25 wt% of said dehydrating agent.

6. A method according to any of claims 4 or 5 wherein said dehydrating agent is selected from the group consisting of activated calcium chloride, activated calcium sulfate, activated zeolite X, activated zeolite A, activated bentonite clay, activated silica gel, and mixtures thereof.

7. A method according to any of claims 4 to 6 which further comprises a dehydrating agent as a distinct element.

8. A method according to any of the preceding claims wherein said moisture-permeable, water-insoluble sachet is further provided in combination with a perfume composition.

9. A method according to claim 8 wherein said sachet, said perfume composition and said dehydrating agent are placed within distinct containers provided with openings so as to allow free circulation of air within said containers.

10. A method according to claim 8 wherein said perfume composition is comprised within said mixture.

11. A method according to claim 8 wherein said perfume composition is loaded onto said sachet.

12. A method according to claim 9 wherein said sachet further comprises a set of usage instructions to direct the user towards selecting, dosing and placing said sachet and/or said perfume composition and/or said dehydrating agent in a suitable manner.

13. A method according to any of the preceding claims for reducing the formation of malodors selected from the group of mold and mildew odors, odors from soap scum and body soil residues, pipe malodors, smoke, cleaning or personal care products malodors, and mixtures thereof, preferably the formation of mold and mildew odors.

14. A method according to any of the preceding claims for reducing the formation of malodors, wherein said confined space is a bathroom and wherein said source of humidity is a bathtub and/or a shower.

15. A method of freshening air in a confined space, said method comprising the step of placing a moisture-permeable, water-insoluble sachet comprising a solid composition comprising a hypohalite bleach and/or a peroxygen bleach in close proximity of source of humidity present within said confined space.

16. The use of a moisture-permeable, water-insoluble sachet comprising a solid composition comprising a hypohalite bleach and/or a peroxygen bleach, for reducing the formation of malodors, preferably the formation of mold and mildew odors.
